Europäisches Patentamt

European Patent Office

Office européen des brevets

(19)

(11) Veröffentlichungsnummer: **0 023 540**
**B1**

(12) **EUROPÄISCHE PATENTSCHRIFT**

(45) Veröffentlichungstag der Patentschrift: **28.12.83**

(21) Anmeldenummer: **80102878.8**

(22) Anmeldetag: **23.05.80**

(51) Int. Cl.³: **C 07 C 17/42,**
**C 07 C 41/58, C 09 K 3/30,**
**C 09 K 15/04**

(54) Gegen Zersetzung durch Wasser stabilisierte chlorierte Kohlenwasserstoffe, gegebenenfalls in Mischung mit Äther, und daraus hergestellte Füllungen für Aerosol-Behälter.

(30) Priorität: **02.06.79 DE 2922700**

(43) Veröffentlichungstag der Anmeldung:
**11.02.81 Patentblatt 81/6**

(45) Bekanntmachung des Hinweises auf die Patenterteilung:
**28.12.83 Patentblatt 83/52**

(84) Benannte Vertragsstaaten:
**AT BE CH DE FR GB IT LI LU NL SE**

(56) Entgegenhaltungen:
**DE - A - 1 768 613**
**US - A - 2 476 554**

(73) Patentinhaber: **HOECHST AKTIENGESELLSCHAFT**
**Postfach 80 03 20**
**D-6230 Frankfurt am Main 80 (DE)**

(72) Erfinder: **Heiskel, Elmar, Dr.**
**Albert-Schweitzer-Strasse 15**
**D-6072 Dreieich (DE)**
Erfinder: **Lendle, Wilhelm, Dr.**
**Hirschpfad 5**
**D-6232 Bad Soden am Taunus (DE)**

**Die Akte enthält technische Angaben, die nach dem Eingang der Anmeldung eingereicht wurden und die nicht in dieser Patentschrift enthalten sind.**

Courier Press, Leamington Spa, England.

# 0 023 540

Gegen Zersetzung durch Wasser stabilisierte chlorierte Kohlenwasserstoffe, gegebenenfalls in Mischung mit Äther, und daraus hergestellte Füllungen für Aerosol-Behälter

Die vorliegende Erfindung betrifft die Stabilisierung von Halogenkohlenwasserstoffen, insbesondere solchen, die bei Raumtemperatur flüssig sind, gegen die Zersetzung in Gegenwart von Wasser. Halogenkohlenwasserstoffe werden unter anderem zur Extraktion, zum Entfetten, zum Reinigen von Metallen und als Lösungsmittel für andere Wirkstoffe benutzt. Bei vielen Einsatzzwecken kommt dabei der Halogenkohlenwasserstoff in Berührung mit Wasser, was zu rascher oder langsamer Zersetzung unter Bildung von Halogenwasserstoff führen kann. Anwesende Metalle, wie z.B. Weißblech, werden dabei in vielen Fällen korrodiert.

Auch für Aerosolabfüllungen werden Halogenkohlenwasserstoffe, vorzugsweise Methylenchlorid und 1,1,1-Trichloräthan, aber auch Fluortrichlormethan, als Lösemittel für eine Vielzahl von Wirkstoffen eingesetzt. In den meisten Fällen wird der Halogenkohlenwasserstoff, z.B. Methylenchlorid, mit anderen organischen Lösemitteln, wie Alkoholen oder Benzinkohlenwasserstoffen, verschnitten. Eine Stabilisierung des Halogenkohlenwasserstoffs in Abwesenheit von Wasser ist im allgemeinen leicht möglich bwz. unnötig. Beispielsweise sind methylenchloridhaltige Insektizid- und Haarsprays in der Regel wasserfrei.

Eine großer Vorteil der genannten Halogenkohlenwasserstoffe ist neben ihrem hohen Lösevermögen für viele Substanzen, daß sie nach den im EG-Raum für Druckgaspackungen verbindlichen gesetzlichen Bestimmungen als nicht brennbar eingestuft sind. Daher kann man durch ihre (Mit-)Verwendung in Sprayformulierungen, insbesondere bei Anwesenheit anderer, brennbarer Komponenten die sicherheitstechnischen Voraussetzungen für nicht deklarationspflichtige Produkte erfüllen. (Die Brennbarkeitsdeklaration ist vorgeschrieben, wenn der gewichtsmäßige Anteil an brennbaren Komponenten in der Gesamtabfüllung 45% erreicht bzw. überschreitet.)

Ein Nachteil der genannten chlorhaltigen Lösemittel ist ihre merkliche Empfindlichkeit gegenüber Wasser. Dies gilt auch für viele andere Halogenkohlenwasserstoffe, insbesondere solche, die nur einen niedrigen Halogenierungsgrad aufweisen und weniger als 4 Fluoratome im Molekül enthalten.

In Gegenwart schon geringer Wasseranteile in einer Aerosolabfüllung, wie sie bereits durch Verschneiden des Chlorkohlenwasserstoffs mit 96 Vol.-%igem Äthanol auftreten können, wird z.B. reines Methylenchlorid, insbesondere im Kontakt mit Metallen in relativ kurzer Zeit (4 bis 8 Wochen) schon so stark hydrolysiert, daß durch den abgespaltenen Chlorwasserstoff Korrosion des metallischen Druckgasbehälters eintritt. Auch das Füllgut wird in den meisten Fällen durch die entstehende Salzsäure chemisch angegriffen und der gesamte Doseninhalt damit unbrauchbar.

Es hat daher nicht an Versuchen gefehlt, durch Zusatz von Inhibitoren Halogenkohlenwasserstoffe, insbesondere die für die Aerosolherstellung besonders wichtigen Verbindungen Methylenchlorid und 1,1,1-Trichloräthan gegen Hydrolyse zu stabilisieren. Beispielsweise wurden Halogenkohlenwasserstoffen, insbesondere Methylenchlorid, Epoxid und/oder sekundäre Amine zugesetzt. Epoxide sind jedoch aus toxikologischer Sicht als bedenklich anzusehen. Sekundäre Amine können in Gegenwart von Nitrit zur Bildung von möglicherweise cancerogenen Nitrosaminen führen. Ferner ist von Nachteil, daß die Wirkung der vorgenannten Stabilisatoren langsam nachläßt und zeitlich begrenzt ist, da diese Stabilisatoren entweder mit dem Halogenkohlenwasserstoff oder dem daraus gebildeten Halogenwasserstoff reagieren. Man hat auch schon N-Methylpyrrol und N-Alkylmorpholin verwendet, um Methylenchlorid (in Abwesenheit von Wasser) gegen die Zersetzung durch Metalle oder Metallsalze zu schützen (GB—PS 932 438). Dies gilt auch für bekannte tertiäre Amine, wie Triäthylamin (US-Patentanmeldung B 370 309).

Es bestand daher die Aufgabe, Halogenkohlenwasserstoffe durch toxikologisch unverdächtige Verbindungen gegen Hydrolyse so zu stabilisieren, daß diese Wirkung sehr lange anhält.

Es wurde nun gefunden, daß sich Halogenkohlenwasserstoffe gegen Zersetzung durch Wasser stabilisieren lassen, wenn ihnen 0,001 bis 10 Gew.-% eines tertiären Amins der Formel

$$RR^1R^2N \qquad\qquad (I)$$

zugesetzt werden, wobei R einen Alkylrest mit 1 bis 10 Kohlenstoffatomen und $R^1$ und $R^2$ unabhängig voneinander einen Isopropyl-, Butyl-(2)-, tert.-Butyl- oder Cyclohexyl-Rest bedeuten. Die Alkylreste können linear, verzweigt oder cyclisch sein.

Bevorzugt sind als Stabilisatoren tertiäre Amine der Formel

$$R_2N{-}C(CH_3)_3 \qquad\qquad (II)$$

wobei R die oben angegebene Bedeutung besitzt.

Vorzugsweise ist R ein Alkylrest mit 1 bis 4 Kohlenstoffatomen, insbesondere der Methyl- oder Äthylrest. Bevorzugt, weil leichter herstellbar, sind tertiäre Amine der Formel I, in denen $R^1$ und $R^2$ identisch sind.

Die zu stabilisierenden Halogenkohlenwasserstoffe sind Methylenchlorid, Fluortrichlormethan und 1,1,1-Trichloräthan.

2

Bevorzugte Konzentrationsbereiche für das erfindungsgemäß eingesetzte tertiäre Amin sind 0,005 bis 5 Gew.-%, insbesondere 0,01 bis 1 Gew.-%.

Es hat sich ferner gezeigt, daß die stabilisierende Wirkung des tertiären Amins in Gegenwart eines Äthers verstärkt wird. Als Äther werden bevorzugt Verbindungen eingesetzt, die nur aus C, H und O bestehen und die frei sind von anderen reaktiven Gruppen. Besonders bevorzugt sind niedermolekulare Alkyläther oder Cycloalkyläther, deren Alkylreste 1 bis 6 C-Atome enthalten. Als wirksam erwiesen sich insbesondere Gehalte an Äther von über 1 Gew.-%. Besonders günstig verhalten sich 5- oder 6-gliedrige Heterocyclen, wie z.B. Tetrahydrofuran, oder unverzweigte Dialkyläther mit 2 bis 6 Kohlenstoffatomen, wie z.B. Diäthyläther, Dimethyläther oder Methyläthyläther.

Gegenstand der Erfindung sind ferner Aerosolbehälter, die als Füllung ein Gemisch aus mindestens einem bei Raumtemperatur und Normaldruck flüssigen Halogenkohlenwasserstoff, mindestens einem verflüssigten, bei Raumtemperatur und Normaldruck gasförmigen Aerosoltreibgas, sowie Wasser, enthalten. In dieser Füllung können noch andere organische Lösungsmittel und Wirkstoffe vorhanden sein. Mit "Wirkstoff" werden in der vorliegenden Anmeldung Stoffe bezeichnet, die nicht als flüssige Halogenkohlenwasserstoffe, organische Lösemittel oder Treibgase anzusehen sind, die aber doch für die Verwendung der Aerosol-Formulierung wesentlich sind. Beispiele hierfür sind Parfüms, Farbstoffe und Bindemittel (in Lacksprays) und Bakterizide. Diese Wirkstoffe haben im allgemeinen nur geringen Einfluß auf die Hydrolyse der vorhandenen Halogenkohlenwasserstoffe. Sie können auch (wie in Kältesprays oder in Abbeizformulierungen auf Basis Methylenchlorid) fehlen.

Besonders interessant sind Aerosolbehälter mit einer Füllung, die

10 — 89.5 Gew.-% flüssigem Halogenkohlenwasserstoff,
0 — 75 Gew.-% andere organische Lösemittel,
0,5 — 25 Gew.-% Wasser,
0 — 35 Gew.-% Wirkstoffe und
10 — 70 Gew.-% verflüssigtes Aerosoltreibgas

enthält, wobei die Füllung durch einen Zusatz von 0,00001 bis 0,1 Gewichtsteil eines tertiären Amins der Formel I, II oder III pro Gewichtsteil flüssigem Halogenkohlenwasserstoff stabilisiert ist.

Besonders wichtig ist die erfindungsgemäße Stabilisierung wenn die obengenannten Aerosol-Formulierung in Aerosoldosen aus Metall abgefüllt werden. In diesem Fall kann der korrosive Angriff auf das Metall (z.B. Weißblech) über lange Zeiträume weitgehend unterdrückt werden.

Die Erfindung wird durch die folgenden Beispiele erläutert:

Beispiele

Versuchsabfüllungen wurden in geschweißte Weißlechaerosoldosen (18 oz) ohne Innenschutzlack gefüllt, mit normalen Aerosolventilen mit blankem Weißblechteller verschlossen und nach Befüllung mit dem Treibmittel stehend bei 40°C gelagert. Nach Ablauf der vorgesehenen Lagerzeiten, im allgemeinen 4, 8 und 12 Wochen, wurden die noch gefüllten Behälter unter den Siedepunkt der eingesetzten Treibmittel abgekühlt, mittels eines Dornes am Dom perforiert und durch Stehenlassen in einem Abzug wieder auf Raumtemperatur gebracht, wobei die Treibmittel-Anteile allmählich verdampften. Die zurückgebliebenen Restbestandteile wurden in Bechergläser geschüttet, die geleerten Dosen aufgesägt. Sowohl die Füllgüter (Füllung ohne Treibmittel) als auch die aufgeschnittenen Weißblechdosen wurden optisch auf Korrosionserscheinungen untersucht. Das Urteil "ohne Befund" bedeutet, daß das Füllgut bzw. Dosenmaterial gegenüber der originären Abfüllung unverändert waren.

Aus den nachfolgenden Tabellen ist der Zusammenhang zwischen Stabilisator für wasserhaltiges Methylenchlorid, Dauer der Lagerung und der Korrosion der Aerosoldosen zu entnehmen. Die Versuche 1 bis 4 stellen Vergleichsversuche dar. Die Versuche 5 bis 7 entsprechen der Erfindung.

TABELLE 1

| Nr. | Zusammensetzung (Gew.-%) | Lagerzeit 4 Wochen | | | Lagerzeit 8 Wochen | | |
|---|---|---|---|---|---|---|---|
| | | Füllgut | Dose | | Füllgut | Dose | |
| | | | Flüssigphase | Gasphase | | Flüssigphase | Gasphase |
| 1 | 20% Methylenchlorid ohne Stabil. 40% Isopropanol 10% Wasser 30% FKW 12 | schmutzigtrüb, mit schwarzem Bodensatz | beginnende Rostbildung | ohne Befund | klar, mit schwarz-braunen schwebenden Teilchen u. dunklem Bodensatz | stark aus-geprägte Rostflecken, besonders im Bereich der Schweißnaht | ohne Befund |
| 2 | 20% Methylenchlorid m. chlorid m. 0,04% Triäthylamin 40% Isopropanol 10% Wasser 30% FKW 12 | klar, mit leichtem dunklem Bodensatz | Rostflekken nur im Schweißnaht-bereich | ohne Befund | klar, leicht gelblich, mit wenig gelb-lichem Bodensatz | Rostflecken im Schweiß-nahtbereich, geringfügig auch am Boden | ohne Befund |
| 3 | 20% Methylenchlorid m. 0.3% Triäthylamin 40% Isopropanol 10% Wasser 30% FKW 12 | klar, mit einem Hauch von Bodensatz | ,, | ohne Befund | klar, sehr wenig weiße Flocken | schwarze, kleine Flecken besonders im Schweiß-nahtbereich | ohne Befund |
| 4 | 20% Methylenchlorid m. 0,04% Triäthylamin 30% Isopropanol 20% Wasser 30% Dimethyläther | klar, mit wenig gelb-lichem Bodensatz | praktisch unverändert | ohne Befund | klar, mit dunklem Bodensatz | beginnende Rostbildung mit kleinen Flecken | ohne Befund |

0023 540

TABELLE 2

| Versuch Nr. | Zusammensetzung (Gew.—%) | Lagerzeit 12 Wochen | | |
|---|---|---|---|---|
| | | Füllgut | Dose | |
| | | | Flüssigphase | Gasphase |
| 1 | 20% Methylenchlorid ohne Stabil. 40% Isopropanol 10% Wasser 30% FKW 12 | klar mit schwarzem Bodensatz | starker Rost am gesamten Körper u. Boden | ohne Befund |
| 2 | 20% Methylenchlorid m. 0,04% Triäthylamin 40% Isopropanol 10% Wasser 30% FKW 12 | klar, mit gelblichem Bodensatz | Rostflecken am Dosenkörper und-boden | ohne Befund |
| 3 | 20% Methylenchlorid m. 0,3% Triäthylamin 40% Isopropanol 10% Wasser 30% FKW 12 | klar, sehr geringer gelblich-weißer Niederschlag | schwarze Flecken im Schweißnahtbereich einzelne am Boden | ohne Befund |
| 4 | 20% Methylenchlorid m. 0,04% Triäthylamin 30% Isopropanol 20% Wasser 30% Dimethyläther | klar, mit schwarzem Bodensatz | Rostflecken am Dosenkörperboden, besonders an der Schweißnaht | ohne Befund |

TABELLE 3

| Nr. | Zusammensetzung (Gew.—%) | Lagerzeit 4 Wochen | | | Lagerzeit 12 Wochen | | |
|---|---|---|---|---|---|---|---|
| | | Füllgut | Dose | | Füllgut | Dose | |
| | | | Flüssigphase | Gasphase | | Flüssigphase | Gasphase |
| 5 | 20% Methylenchlorid mit 0,3% N-Äthyldiiso-propylamin 45% Isopropanol 5% Wasser 30% FKW 12 | ohne Befund | ohne Befund | ohne Befund | wasserklar, geringer dkl. brauner Bodensatz | *Ein* Korrosions-flek an d. Schweißnaht, einige am Bodenrand | ohne Befund |
| 6 | 20% Methylenchlorid mit 0,04% N-Äthyldiiso-propylamin 45% Isopropanol 5% Wasser 30% Dimethyläther | ohne Befund | ohne Befund | ohne Befund | klar, schwach gelbstichig | ohne Befund | ohne Befund |
| 7 | 20% Methylenchlorid mit 0.3% N-Äthyldicyclo-hexylamin 45% Isopropanol 5% Wasser 30% Dimethyläther | ohne Befund | ohne Befund | ohne Befund | ohne Befund | ohne Befund | ohne Befund |

# 0 023 540

## Patentansprüche

1. Halogenkohlenwasserstoff ausgewählt aus der Gruppe bestehend aus Methylenchlorid, Trichlorfluormethan und 1.1.1-Trichloräthan, stabilisiert gegen die Zersetzung durch Wasser, dadurch gekennzeichnet, daß als Stabilisator 0,001 bis 10 Gew.-% eines tertiären Amins der Formel

$$RR^1R^2N$$

eingesetzt wird, wobei R einen Alkylrest mit 1 bis 10 Kohlenstoffatomen und $R^1$ und $R^2$, unabhängig voneinander, einen Isopropyl-, Butyl-(2)-, tert.-Butyl- oder Cyclohexylrest bedeuten.

2. Halogenkohlenwasserstoff gemäß Anspruch 1, dadurch gekennzeichnet, daß das tertiäre Amin der Formel

$$RR^1_2N$$

besitzt.

3. Halogenkohlenwasserstoff gemäß Anspruch 1, dadurch gekennzeichnet, daß R einen Alkylrest mit 1 bis 4 Kohlenstoffatomen bedeutet.

4. Halogenkohlenwasserstoff nach einem der Ansprüche 1 bis 3, dadurch gekennzeichnet, daß 0,01 bis 1 Gew.-% des tertiären Amins als Stabilisator eingesetzt werden.

5. Halogenkohlenwasserstoff/Äther-Gemisch, in dem der Halogenkohlenwasserstoff aus Methylenchlorid, Trichlorfluormethan oder 1.1.1-Trichloräthan besteht, mit einem Gewichtsverhältnis Halogenkohlenwasserstoff/Äther von 1:2 bis 100:1, dadurch gekennzeichnet, daß es als Stabilisator 0,001 bis 10 Gew.% des tertiären Amins gemäß Anspruch 1 enthält.

6. Verwendung von tertiären Aminen gemäß Anspruch 1 zum Stabilisieren von Methylenchlorid, Trichlorfluormethan oder 1.1.1-Trichloräthan gegen Zersetzung durch Wasser.

7. Verwendung von tertiären Aminen gemäß Anspruch 1 zum Stabilisieren eines Halogenkohlenwasserstoff/Äther-Gemisches, in dem der Halogenkohlenwasserstoff aus Methylenchlorid, Trichlorfluormethan oder 1.1.1-Trichloräthan besteht, gegen Zersetzung durch Wasser.

8. Aerosolbehälter mit einer Füllung bestehend aus

10 — 89.5 Gew.-% Methylenchlorid, Trichlorfluormethan und/oder 1.1.1-Trichloräthan
0 — 75 Gew.-% anderen organischen Lösemitteln,
0,5 —25 Gew.-% Wasser,
0 —35 Gew.-% Wirkstoffen und
10 — 70 Gew.-% verflüssigtem Aerosoltreibgas,

dadurch gekennzeichnet, daß diese Mischung 0,00001 bis 0,1 Gewichtsteile eines tertiären Amins von Anspruch 1 pro Gewichtsteil Halogenkohlenwasserstoff enthält.

9. Aerosolbehälter gemäß Anspruch 8, dadurch gekennzeichnet, daß der Behälter aus Metall gefertigt ist.

## Revendications

1. Hydrocarbure halogénè choisi parmi le chlorure de méthylène, le trichlorofluorométhane et le 1,1,1-trichloréthane, stabilisé contre la décomposition par l'eau, caractérisé en ce qu'il est stabilisé par une addition de 0,001 à 10% en poids d'une amine tertiaire de formule:

$$RR^1R^2N$$

le symbole R désignant un alkyle en $C_1$ à $C_{10}$ et $R^1$ et $R^2$ désignant chacun, indépendamment l'un de l'autre, un radical isopropyle, butyle-(2), tert-butyle ou cyclo-hexyle.

2. Hydrocarbure halogéné selon la revendication 1, caractérisé en ce qu'il est additionné d'une amine tertiaire de formule:

$$RR^1_2N$$

3. Hydrocarbure halogéné selon la revendication 1, caractérisé en ce que l'alkyle R de l'amine tertiaire est un alkyle en $C_1$ à $C_4$.

4. Hydrocarbure halogéné selon l'une quelconque des revendications 1 à 3, caractérisé en ce qu'il est additionné de 0,01 à 1% en poids de l'amine tertiaire comme stabilisant.

5. Mélange d'un hydrocarbure halogéné et d'un éther, dont l'hydrocarbure halogéné est le chlorure de méthylène, le trichlorofluorométhane ou le 1,1,1-trichloréthane, dans un rapport pondéral de l'hydrocarbure halogéné à l'éther compris entre 1:2 et 100:1, mélange caractérisé en ce qu'il contient comme stabilisant 0,001 à 10% en poids d'une amine tertiaire selon la revendication 1.

6. Utilisation d'amines tertiaires selon la revendication 1 pour stabiliser le chlorure de méthylène, le trichlorofluorométhane ou le 1,1,1-trichloréthane contre leur décomposition par l'eau.

7. Utilisation d'amines tertiaires selon la revendication 1 pour stabiliser contre sa décomposition par l'eau un mélange d'un hydrocarbure halogéné et d'un éther, mélange dont l'hydrocarbure halogéné est le chlorure de méthylène, le trichlorofluorométhane ou le 1,1,1-trichloréthane.

8. Récipient d'aérosol garni d'un mélange de

10 à 89,5% en poids de chlorure de méthylène, de trichlorofluorométhane, et/ou de 1,1,1-trichloréthane,

0 à 75% en poids d'autres solvants organiques,

0.5 à 25% en poids d'eau,

0 à 35% en poids de matières actives et

10 à 70% en poids d'un gaz propulseur (gaz de projection) pour aérosols liquéfié,

caractérisé en ce que ce mélange contient aussi 0,00001 à 0,1 partie en poids d'une amine tertiaire selon la revendication 1 par partie en poids de l'hydrocarbure halogéné.

9. Récipient d'aérosol selon la revendication 8, caractérisé en ce qu'il est un récipient métallique.

**Claims**

1. Halogenated hydrocarbon selected from the group consisting of methylene chloride, trichlorofluormethane and 1.1.1-trichloroethane, stabilized against decomposition by water, which comprises as stabilizer from 0.001 to 10% by weight of a tertiary amine of the formula

$$RR^1R^2N$$

with R being alkyl with 1 to 10 carbons and $R^1$ and $R^2$, independent from one another, being isopropyl, butyl-(2), tertiary butyl or cyclohexyl.

2. Halogenated hydrocarbon according to claim 1, comprising the tertiary amine of the formula

$$RR^1_2N$$

3. Halogenated hydrocarbon according to claim 1, wherein R is an alkyl with 1 to 4 carbon atoms.

4. Halogenated hydrocarbon according to any one of the claims 1 to 3, comprising as stabilizer from 0.01 to 1% by weight of the tertiary amine.

5. Halogenated hydrocarbon/ether mixture in which the halogenated hydrocarbon is selected from the group consisting of methylene chloride, trichlorofluoromethane and 1.1.1-trichloroethane with a weight ratio of halogenated hydrocarbon/ether of from 1:2 to 100:1, comprising as stabilizer the mixture from 0.001 to 10% by weight of the tertiary amine according to claim 1.

6. Use of tertiary amines according to claim 1 for stabilizing methylene chloride, trichlorofluormethane or 1.1.1-Trichloroethane against decomposition by water.

7. Use of tertiary amines according to claim 1 for stabilizing mixture of halogenated hydrocarbons and ether in which the halogenated hydrocarbon consists of methylene chloride, trichlorofluormethane or 1.1.1-trichloroethane against decomposition by water.

8. Aerosol bottle containing a charge consisting of

10 — 89.5 % by weight of methylene chloride, trichlorofluormethane and/or 1.1.1-trichloroethane,

0 — 75% by weight of other organic solvents,

0 — 25% by weight of water,

0 — 35% by weight of active ingredients and

10 — 70% by weight of liquefied aerosol propellant,

wherein the charge contains from 0.00001 to 0.1 part of weight of a tertiary amine of claim 1 per part by weight of halogenated hydrocarbon.

9. Aerosol bottle according to claim 8, wherein the bottle is made from metal.